# EUROPEAN PATENT APPLICATION

(11) **EP 2 223 909 A1**
(43) Date of publication of application: **01.09.2010**
(21) Application number: 10164317.9
(22) Date of filing: 27.08.2008
(51) Int. Cl.: C07C 235/06, C07C 67/31, C07C 67/313, C07C 69/675, C07D 239/42, C07F 9/535

(54) **Process for preparing pentanoic diacid derivatives**

(30) Priority: 28.08.2007 EP 07016872
(62) Divisional of application: 08801731.4
(71) Applicant: Ratiopharm GmbH, 89079 Ulm (DE)
(72) Inventor: Börner, Armin, 18059 Rostock (DE); König, Gerd, 08056 Zwickau (DE); Andrushko, Vasyl, 76137 Karlsruhe (DE); Andrushko, Natalia, 76137 Karlsruhe (DE)
(74) Representative: Teipel, Stephan

(57) **Abstract**

The present invention relates to a process for preparing pentanoic diacid derivatives useful for preparing pyrimidine derivatives, in particular as intermediates useful for preparing pyrimidine derivatives of a class that is effective at inhibiting the biosynthesis of cholesterol in humans, such as HMG-CoA reductase inhibitors, e.g. rosuvastatin.

## Description

The present invention relates to a process for preparing pentanoic diacid derivatives (glutaric acid derivatives) as intermediates useful for preparing pyrimidine derivatives of a class that is effective at inhibiting the biosynthesis of cholesterol in humans, and more particularly to improved synthetic methods for preparing rosuvastatin.

It is known that certain 3,5-dihydroxy heptanoic acid derivatives are competitive inhibitors of the 3-hydroxy-3-methyl-glutaryl-coenzyme A ("HMG-CoA"). HMG-CoA is a key enzyme in the biosynthesis of cholesterol in humans. Its inhibition leads to a reduction in the rate of biosynthesis of cholesterol. The first HMG-CoA inhibitor to be described is compactin ([1*S*-[1α(*R**),7β,8β(2*S**,4*S**),8α[β]]-1,2,3,7,8a-hexahydro-7-methyl-8-[2-(tetrahydro-4-hydroxy-6-oxo-2*H*-pyran-2-yl)ethyl]-1-naphthalenyl 2-methylbutanoate), which was isolated from cultures of *Penicillium* in 1976. In 1987, lovastatin ([1*S-*[1α(*R**),3α,7β,8β(2*S**,4*S**),8αβ]]-1,2,3,7,8,8a-hexahydro-3,7-dimethyl-8-[2-(tetrahydro-4-hydroxy-6-oxo-2*H*-pyran-2-yl)ethyl]-1-naphthalenyl 2-methylbutanoiate) became the first HMG-CoA reductase inhibitor approved by the Food and Drug Administration (FDA) for treatment of hypercholesterolemia. Both compactin and lovastatin are derived from bacterial cultures. Two other naturally-derived HMG-CoA reductase inhibitors, simvastatin and pravastatin are structurally related to compactin and lovastatin.

Another known HMG-CoA reductase inhibitor which can be used for the treatment of, inter alia, hypercholesterolemia and mixed dyslipidemia is rosuvastatin. Rosuvastatin has the chemical name(*E*)-7-[4-(4-fluorophenyl)-6-isopropyl-2-[methyl(methylsulfonyl)-amino]pyrimidin-5-yl](3*R*,5*S*)-3,5-dihydroxyhept-6-enoic acid and the structural formula

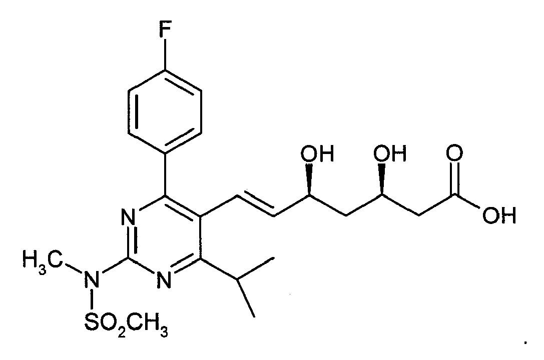

Rosuvastatin calcium is marketed under the trademark CRESTOR^{™}.

In contrast to compactin, lovastatin, simvastatin and pravastatin, there is no known fermentation culture that produces rosuvastatin. It must therefore be synthesized by traditional synthetic methods.

A number of processes for the synthesis of rosuvastatin and derivatives thereof are known. Some of the processes are concerned with the synthesis of the 3,5-dihydroxy hepten-6-oic acid side chain of the pyrimidine ring while others are concerned with the formation of the pyrimidine ring or the linkage of the side chain to the pyrimidine ring.

In the synthesis of rosuvastatin for the formation of the double bond in the C7 side chain, the application of the Wittig reaction has long been found to be advantageous (cf. Scheme 1).

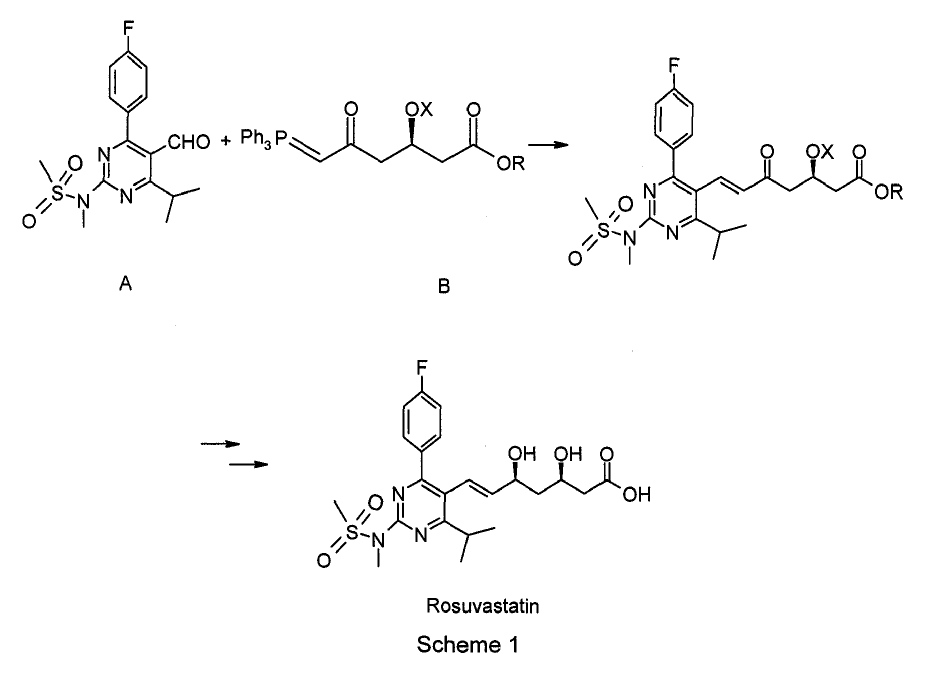

US 5,260,440 discloses the reaction of methyl(3*R*)-3-(*tert*-butyldimethylsilyloxy)-5-oxy-6-triphenylphosphoranyliden hexanoic acid derivatives (cf. compound B of Scheme 1, X = *t*-butyldimethylsiloxy) with 4-(4-fluorophenyl)-6-isopropyl-2-(*N*-methyl-*N-*methylsulfonylamino)-5-pyrimidine-carboxaldehyd (cf. compound A of Scheme 1), followed by a deprotection step, a reduction step and a hydrolysis step to obtain rosuvastatin.

WO 00/49014 discloses the synthesis of rosuvastatin *via* a Wittig reaction using a Wittig reagent which comprises the pyrimidine core of the rosuvastatin molecule.

WO 03/087112 also discloses the synthesis of rosuvastatin using a Wittig reaction.

It is known in the prior art that it is desirable to apply the Wittig reagents, i.e. compound B of Scheme 1 above, in the Wittig reaction already with the correct stereo configuration in order to obtain a product which can easily be converted into rosuvastatin.

To obtain the Wittig reagent B with the desired stereo configuration it is also known to start from a 3-hydroxy(protected)dipentanoic acid derivative (4) as depicted in the following scheme 2:

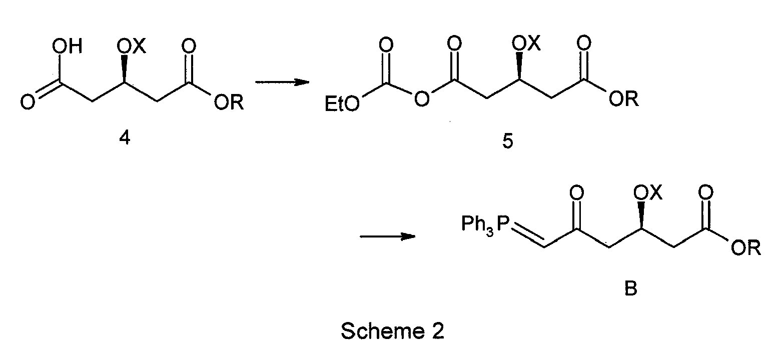

According to the scheme 2, compound (4) is converted according to known procedures, as e.g. described in WO 03/087112 A1, WO 2006/091771 A2, or EP 554 455 A1, the disclosure of which documents is enclosed by reference herein, to obtain the Wittig reagent B.

The preparation of compound (4) by resolution of a racemate or asymmetric synthesis is known. Conventional known routes to prepare enantiomerically pure dipentanoic derivatives such as compound 4, however, are typically disadvantageous, especially for industrial preparation scales, as e.g. special expensive reagents or enzymes are required. Further the achieved enantiomeric excesses are often unsatisfactory.

Therefore, there is still a need for further methods of synthesizing pentanoic diacid derivatives such as compound (4) and in particular pentanoic diacid derivatives as intermediates for the preparation of pyrimidine derivatives, such as rosuvastatin, in particular such which are industrially applicable and provide sufficient enantiomeric excesses.

It has now been found that several prior art problems can surprisingly be overcome by a modified synthesis of certain dipentanoic acid derivatives which further may be used as intermediates in the reaction of these pentanoic acid derivatives to prepare Wittig reagents, in particular such as compound B and derivatives thereof to be applied in the synthesis of rosuvastatin.

In particular, it has been found that said dipentanoic acid derivatives can be synthesized according to the following reaction scheme 3

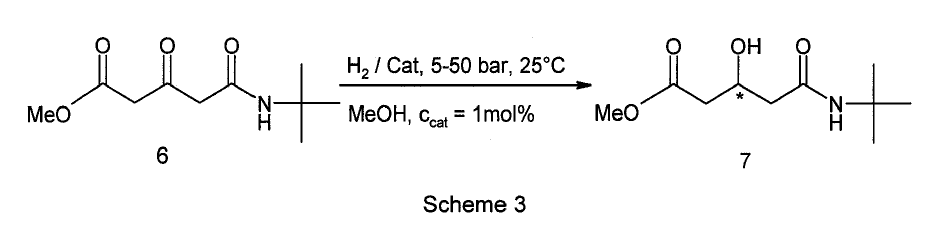

In particular it was surprisingly found that 3-oxopentanoic diacid derivatives can be catalytically reduced into the corresponding 3-hydroxy-pentanoic diacid derivatives with excellent enantiomeric excesses (ee).

Alternatively, said dipentanoic derivatives can be synthesized according to the following reaction scheme 4 or reaction scheme 4':

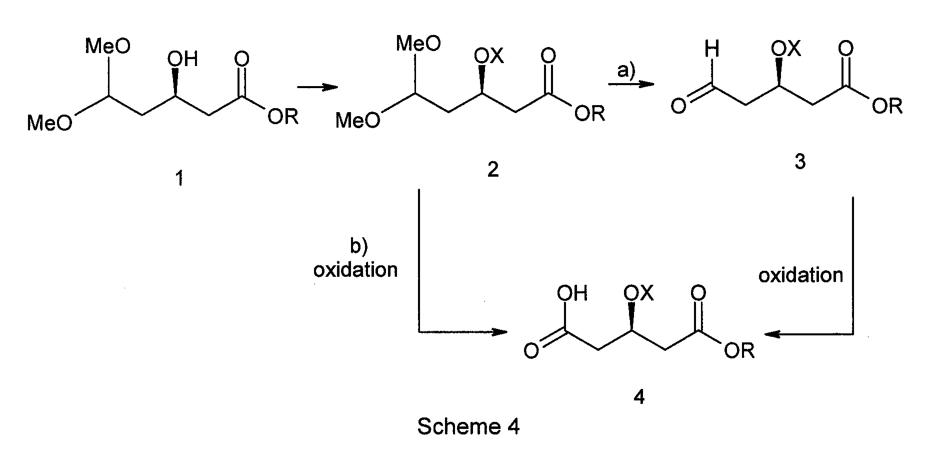

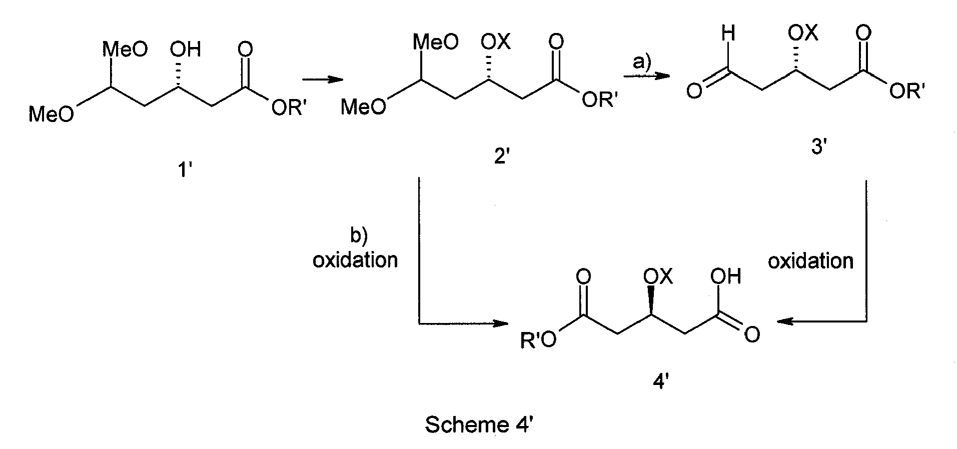

In particular it was surprisingly found that 3-hydroxy pentanoic diacid derivatives can be obtained from 5,5-dimethoxy-3-hydroxy-pentanoate derivatives (1 or 1'), which are obtainable by catalytic hydrogenation of 5,5-dimethoxy-3-oxopentanoate derivatives. Typically the hydroxy group of said 5,5-dimethoxy-3-hydroxy-pentanoate derivatives is protected. This is followed by a) hydrolysis of the 5,5-dimethoxy-3-hydroxy(protected)-pentanoate derivatives (2 or 2') to obtain the corresponding aldehyde (3 or 3') and oxidation or b) direct oxidation of (2 or 2') to obtain a corresponding 3-hydroxy(protected)dipentanoic acid derivative (4 or 4').

Compound (4) can be obtained from compound (4') by protection of the free carboxylic acid group (i.e. -COOH -> COOR) of compound (4') and deprotection of the protected carboxylic acid group (i.e. -COOR' -> COOH) of compound (4').

Therefore, the present invention relates to a process for the preparation of a compound of the formula I 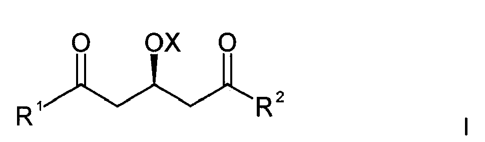 or a salt thereof, wherein X is H or a hydroxy protecting group, and R¹ and R² are independently selected from OH, OR³, wherein R³ is a carboxyl protecting group, or NR⁴R⁵, wherein R⁴ and R⁵ are independently H or an amido protecting group,
which process comprises the steps of
a) hydrogenating a compound of the formula II 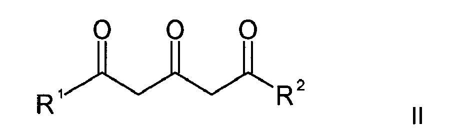 or a salt thereof, wherein R¹ and R² are defined as above,
   to obtain a compound of the formula III 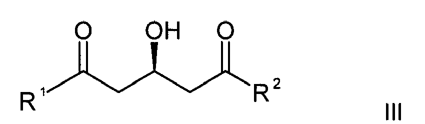 or a salt thereof, wherein R¹ and R² are defined as above, or
b) reacting a compound of the formula IV
   or a salt thereof, 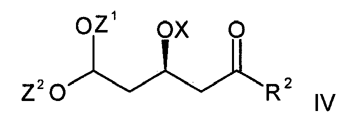 wherein Z¹ is a hydroxy protecting group, Z² is a hydroxy protecting group, and X and R² are defined as above, to obtain a compound of formula V
   or a salt thereof, 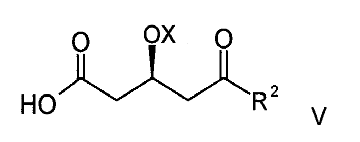 wherein X and R² are defined as above, and
c) optionally converting the residues of the compound of the formula III obtained from step a) or the residues of the compound of the formula V obtained from step b) to obtain the compound of the formula I.

The compound of formula I prepared by the process of the present invention is intended as intermediate for the preparation of the Wittig reagent, such as compound B of scheme 2 above, for the preparation of pyrimidine derivatives, in particular rosuvastatin.

Residues R¹ and R² within the compounds of the present invention are independently selected from OH, OR³ and NR⁴R⁵, wherein R³ is a carboxyl protecting group, R⁴ and R⁵ are independently H or an amido protecting group.

As protecting groups for the optionally protected hydroxy groups, the optionally protected carboxyl groups and the optionally protected amido groups usual protecting groups known to the person skilled in the art may be used. Suitable protecting groups are exemplified in WO 03/044011, the disclosure of which is incorporated by reference herein.

Preferred protecting groups for Z¹ Z², Z¹', Z²', Y', X, X', R³, R⁴ and R⁵ are alkyl, aryl and aralkyl, such as straight, branched or cyclic C₁₋₁₀ alkyl, preferably C₁₋₆ alkyl, more preferably methyl, ethyl, isopropyl, or *tert*-butyl. Aryl can be for example phenyl or naphthyl. Aralkyl can be for example aryl such as phenyl or naphthyl linked via a C₁₋₁₀ alkyl, preferably C₁₋₆ alkylene, such as benzyl. More preferred X and/or X' is a tri(C₁₋₆ alkyl)silyl or a diarylalkylsilyl, even more preferred a trimethylsilyl, a *tert*-butyldimethylsilyl or a diphenyl(*tert*-butyl)silyl group. Most preferred Z¹, Z², Z¹' and Z²' are methyl groups.

In one preferred embodiment of the present invention R¹ is OR³ and R³ is alkyl, aryl or aralkyl, preferably R³ is a C₁₋₆ alkyl group, most preferred R³ is a methyl, ethyl or *tert-*butyl group, R² is NR⁴R⁵ and R⁴ and R⁵ are independently alkyl, aryl or an aralkyl group, preferably R⁴ is a C₁₋₆ alkyl group and R⁵ is H, most preferred R⁴ is a *tert*-butyl group and R⁵ is H, and X is H or a hydroxy protecting group, in particular X is H or a SiPh₂t-Bu group, whereby "Ph" means a phenyl group.

In one preferred embodiment of the present invention R¹ and R² are OR³ and R³ is alkyl, aryl or aralkyl, preferably R³ is a C₁₋₆ alkyl group, X is H or a hydroxy protecting group and Z¹ and Z² are a hydroxy protecting group.

Process step c) of the process of the present invention typically comprises steps of converting in the compound of the formula III obtained from step a) or in the compound of the formula V obtained from step b) any carboxyl group into an amido group or any amido group into a carboxyl group, optionally protecting or deprotecting the carboxyl groups, the amido groups and/or the hydroxy groups and/or optionally converting the resulting compound into a salt thereof.

How to obtain the compound of formula II is known in the art.

Preferably the compounds of the formula IV can be synthesized according to the following scheme 5:

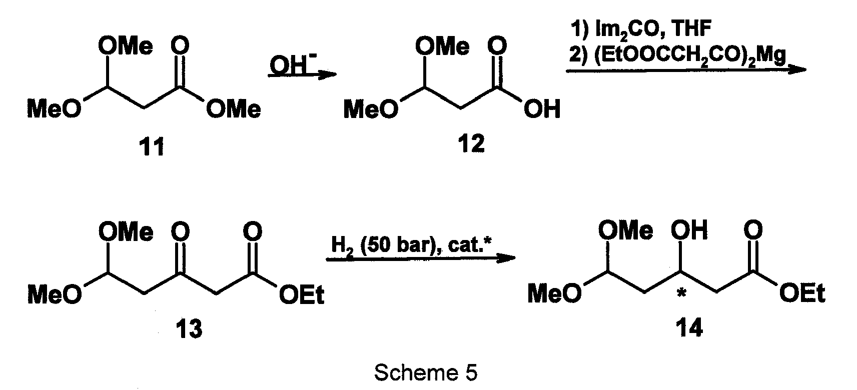

The advantage of this sequence is the high enantioselectivity which can be achieved synthesizing the C5 intermediate (14). In particular the reduction of a β-keto ester containing an acetal group (confer compound (13)) can be performed with excellent enantiomeric excess (ee).

Therefore, the compound of the formula IV to be used in the process of the present invention is preferably obtained by the process for the preparation of a compound of the formula IV 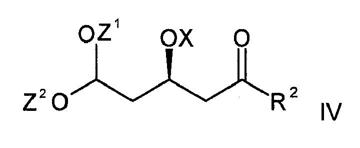 or a salt thereof, wherein Z₁ is a hydroxy protecting group, Z₂ is a hydroxy protecting group, X is H or a hydroxy protecting group, and R² is defined as above, which process comprises the steps of
A) reacting a compound of the formula XII 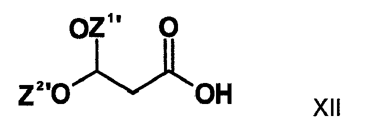 wherein Z¹' is a hydroxy protecting group and Z²' is a hydroxy protecting group, with N,N'-carbonyldiimidazole (Im₂CO) and a compound of the formula XIII 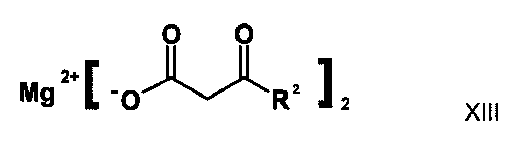 wherein R² is defined as above, to a compound of the formula XIV 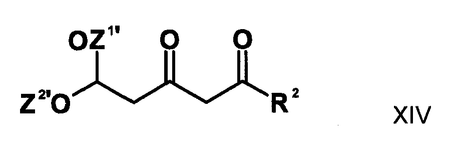 wherein Z¹', Z²' and R² are defined as above,
B) reducing the compound of the formula XIV to obtain a compound of the formula XV 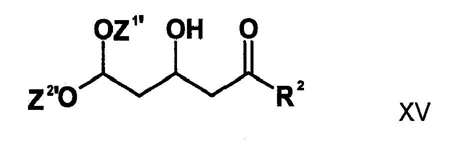 wherein Z¹', Z²' and R² are defined as above, and
C) optionally deprotecting the hydroxy groups and/or the carboxy group of the compound of the formula XV, optionally protecting the free hydroxy group of the compound of the formula XV and/or optionally converting the resulting compound into a salt thereof.

In step A) residue R² is preferably OR³, and R³ is defined as described above.

The reduction step B) in the process to prepare a compound of the formula IV, which preferably is a hydrogenation step, can be carried out under chiral or achiral conditions. Under achiral conditions a mixture of the two enantiomers of the compound of formula IV is obtained. If it is desired to obtain the compound in its chiral form comprising mainly only one of its enantiomers, the reduction step B) can be carried out under chiral conditions, for example in the presence of a chiral catalyst, e.g. as described for step a). Preferably the hydrogenation is carried out under homogenous chiral enantioselective conditions, e.g. as described for step a).

Suitable catalysts in step B) may be selected from iridium, rhodium and ruthenium complexes, such as Ru((*R*)-BINAP) = (*R*)-2,2'-Bis(diphenylphosphanyl)-1,1'-binapthyl and Ru((*R*)-Tol-BINAP) = (*R*)-2,2'-Bis(di-*p*-tolylphosphanyl)-1,1'-binapthyl, which are preferred. For example Ru-BINAP catalysts are described in Tetrahedron Lett. 1991, 32, 4163 and WO 95/18784, the content of these documents is incorporated herein by reference.

Preferably in step B) the ligand in the Rh, Rn and lr complexes should be chiral. For example the following ligands can further be used for Rh, Ru and lr catalyzed asymmetric hydrogenation:
(*R*)-NORPHOS - (2*R*,3*R*)-(-)-2,3-bis(diphenylphosphanyl)-bicydo[2.2.1]hept-5-ene.
(*R,R*)-CHIRAPHOS = (2*R*,3*R*)-(-)Bis(diphenylphosphanyl)butane
(*R,R*)-DEGUPHOS = (3*R*,4*R*)-(+)-1-Benzyl-3,4-bis(diphenylphosphanyl)pyrrolidine
(*R,R*)-Me-DUPHOS = (-)-1,2-Bis[(2*R*,5*R*)-2,5-dimethylphospholanyl]benzene
(*R,R*)-Et-DUPHOS = (-)-1,2-Bis[(2*R*,5*R*)-2,5-diethylphospholanyl]benzene
(*R,R*)-DIPAMP = (1*R*,2*R*)-Bis[(2-methoxyphenyl)phenylphosphanyl]ethane
(*R,R*)-bdpch = (1*R*,2*R*)-(*trans*)-1,2-Bis(diphenylphosphanyloxy)cyclohexane
(*S,S*)-DIOP = (4*S*,5*S*)-(+)-4,5-Bis(diphenylphosphanylmyrthyl)-2,2-dimethyl-1,3-dioxolane
Ph-β-Glup = Phenyl 4,6-*O*-benzylidene-2,3-bis(*O*-diphenylphosphanyl)-β-D-glucopyranoside.

In the above ligands the configuration is given as example only. It is understood that the skilled person will choose the configuration of the ligand such that the obtained hydrogenated product has the desired configuration.

The hydrogenation in step B) can be carried out under usual conditions, for example in the presence of hydrogen at about room temperature, such as 25°C, under elevated pressure for example in the range of 20 - 80 bar, preferably 30 - 70 bar and in particular 40 - 50 bar. The reaction can be carried out in a suitable solvent, preferably a polar protic solvent, in particular a C₁₋₆ alcohol, such as methanol, which is preferred, or ethanol, or an ester, such as ethyl acetate. The reaction can be carried out until hydrogen consumption is finished.

However, when trying to synthesize the required tert-butyl ketoester of the formula XIV starting from a compound of formula XIII using traditional Knoevenagel reaction in the last step, while the desired ketoester was formed it was contaminated with about 15% tert-butyl acetoacetate. No reasonable method of purification was found.

While the mono tert-butyl malonate required in the preparation of the intermediate compound of the formula XIII is commercially available, this compound is expensive and therefore not feasible for use in a commercial process. Therefore, the inventors tried to obtain the required mono tert-butyl malonate by reaction of Meldrum's acid with tert-butanol according to D.W. Brooks, et al., Tedrahedron Lett., 1984, 25, 4623-26 and S. Masayuki, et al., Tetrahedron Lett., 1997, 38, 4623-26 (see reaction scheme 6). It was found that refluxing of Meldrum's acid in excess of tert-butanol gives the desired mono tert-butyl malonate as the main product but also the cyclic compound depicted in scheme 6 as the side product in a ratio of about 30:1.

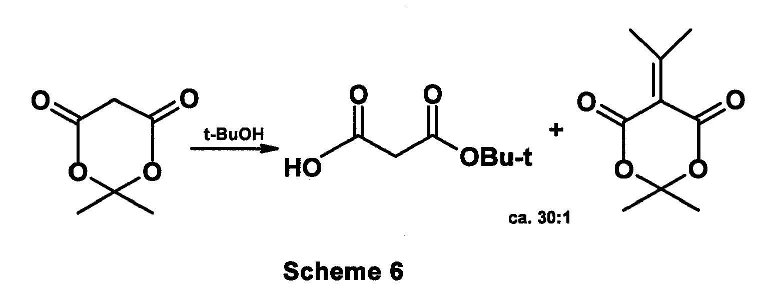

Purification of mono tert-butyl malonate by distillation was unsuccessful since disproportionation of the material to di-tert-butyl malonate and malonic acid was observed.

Surprisingly it was found that a fast purification of mono tert-butyl malonate via its ammonium salt is possible. This salt can be isolated with a yield of 86.7%. Treatment of this salt with hydrochloric acid affords pure (NMR) mono tert-butyl malonate with a yield of 94.3%.

In the process of the present invention for the preparation of the compound of the formula IV reaction step A) can be carried out under conditions as described for example in T. Honore, et al., Eur. J. Med. Chem. Chim. Ther., 1978, 13, 429-34.

The hydrogenation step (a) in the process of the present invention can be carried out under chiral or achiral conditions. Under achiral conditions a mixture of the two enantiomers of the compound of formula I is obtained. If it is desired to obtain a compound in its chiral form comprising mainly only one of its enantiomers, the hydrogenation step (a) can be carried out under chiral conditions, e.g. in the presence of a chiral catalyst. Preferably the hydrogenation is carried out under homogeneous chiral enantioselective conditions.

Suitable catalysts may be selected from iridium, rhodium and ruthenium complexes, preferably rhodium and ruthenium complexes.

Preferably the ligand in the Rh, Ru and lr complexes should be chiral. For example, the following ligands can be used for Rh, Ru and lr catalysed asymmetric (enantioselective) hydrogenation:
(*R*)-NORPHOS = (2*R*,3*R*)-(-)-2,3-Bis(diphenylphosphanyl)-bicyclo[2.2.1]hept-5-ene
(*R,R*)-CHIRAPHOS = (2*R*,3*R*)-(-)Bis(diphenylphosphanyl)butane
(*R,R*)-DEGUPHOS = (3*R*,4*R*)-(+)-1-Benzyl-3,4-bis(diphenylphosphanyl)pyrrolidine
(*R,R*)-Me-DUPHOS = (-)-1,2-Bis[(2*R*,5*R*)-2,5-dimethylphospholanyl]benzene
(*R,R*)-Et-DUPHOS = (-)-1,2-Bis[(2*R*,5*R*)-2,5-diethylphospholanyl]benzene
(*R,R*)-DIPAMP = (1*R*,2*R*)-Bis[(2-methoxyphenyl)phenylphosphanyl]ethane
(*R,R*)-bdpch = (1*R*,2*R*)-(*trans*)-1,2-Bis(diphenylphosphanyloxy)cyclohexane
(*S,S*)-DIOP = (4*S*,5*S*)-(+)-4,5-Bis(diphenylphosphanylmyrthyl)-2,2-dimethyl-1,3-dioxolane
Ph-β-Glup = Phenyl 4,6-*O*-benzyylidene-2,3-bis(*O*-diphenlphosphanyl)-β-D-glucopyranoside.

Most preferred are BINAP containing catalysts, such as (*S*)-(-)-[Ru(BINAP)(*p-*cymene)CI]CI, (*R*)-(+)-[Ru(BINAP)(*p*-cymene)CI]CI or (*R*)-[Ru(BINAP)]CI₂. BINAP means 2,2'-Bis(diphenylphosphino)-1,1'-binaphtyl. The catalysts can be used as solvent additives, e.g. as (*R*)-[Ru(BINAP)]Cl₂xImHxDMF.

In the above ligands the configuration is given as example only. It is understood that the skilled person will choose the configuration of the ligand such that the obtained hydrogenated product has the desired configuration.

The hydrogenation can be carried out under usual conditions, for example in the presence of hydrogen at about room temperature, such as at about 25°C, under elevated pressure for example in the range of about 2 to about 80 bar, preferably about 5 to about 50 bar and in particular about 5 to about 25 bar. The reaction can be carried out in a suitable solvent, preferably a polar protic solvent, in particular a C₁₋₆ alcohol, such as methanol, which is preferred, or ethanol, or an ester, such as ethyl acetate. The reaction can be carried out until hydrogen consumption is finished.

The reaction step (b) in the process of the present invention can be carried our under application of known chemical synthesis steps. Such known chemical synthesis steps comprise hydrolysis steps, reduction steps, oxidation steps and/or protecting and deprotecting steps. Generally any sequence of reaction steps useful for converting a protected acetal group, such as the group -CH(OZ¹)(OZ²), into the corresponding acid, ester or amido group, respectively, of the compound of the formula I, i.e. -C(=O)R¹.

In a preferred embodiment of the process of the present invention, the reaction step (b) comprises a step of oxidating the compound of the formula IV 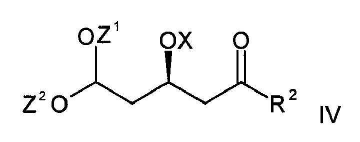 or a salt thereof,
wherein Z¹ is a hydroxy protecting group, Z² is a hydroxy protecting group, and X and R² are defined as above, to obtain a compound of the formula V 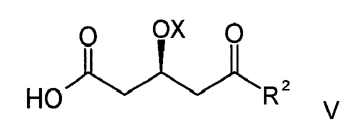 or a salt thereof,
wherein X and R² are defined as above.

In this embodiment, wherein the reaction step (b) comprises a step of oxidating the compound of the formula IV, the oxidation may be carried out using reagents and reaction conditions as known in the art, in particular for oxidating a protected acetal group, such as a -CH(OZ¹)(OZ²) group into a carboxyl group. The oxidation can e.g. be carried out with Jones' oxidation reagents at about 0°C in a solvent suitable for Jones' oxidation, such as acetone. Under such reaction conditions both cleavage of the acetate moiety, i.e. deprotecting the acetate group, and oxidation of the resulting aldehyde group can be performed in an one-pot reaction.

In a further preferred embodiment of the present invention the reaction step (b) comprises the step of hydrolizing the compound of the formula IV 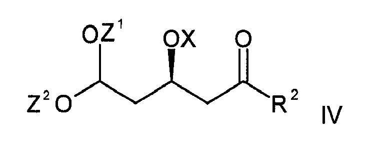 or a salt thereof,
wherein Z¹ is a hydroxy protecting group, Z² is a hydroxy protecting group, and X and R² are defined as above, to obtain a compound of the formula VI 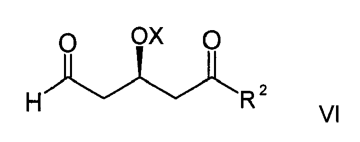 or a salt thereof,
wherein X and R² are defined as above, and optionally directly following the hydrolizing step, the step of oxidating the compound of the formula Vl to obtain a compound of the formula V 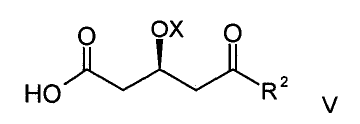 or a salt thereof,
wherein X and R² are defined as above.

In this embodiment of the process of the present invention, wherein the reaction step (b) comprises a hydrolizing step and an oxidation step, preferably the oxidation step is conducted directly following the hydrolizing step. The hydrolizing step may be conducted following known hydrolizing reaction conditions, in particular such for hydrolizing an protected acetal group to obtain the unprotected aldehyde group. Such hydrolizing step, i.e. cleavage of the acetal moiety, can be performed in suitable solvents, such as acetone/H₂O, in the presence of preferably catalytic amounts of a suitable acid, such as p-toluenesulfonic acid hydrate. The aldehyde obtained from the hydrolizing step may be purified or directly used in the oxidation step, which preferable directly follows the hydrolizing step. The oxidation step may be carried out as described above, when the compound of the formula IV is directly oxidized to obtain a compound of the formula V. Generally, many different oxidation procedures, particular such for oxidizing an aldehyde group to obtain a corresponding carboxyl group may be applied, which are known in the art. Most preferably a Jones' oxidation may be applied, e.g. at about 0°C in a suitable solvent, such as acetone. How to conduct the Jones' oxidation is known in the art, and typically comprises the application of a solution of chromium trioxide (Cr0₃) in concentrated sulfuric acid (H₂SO₄) as oxidating agent in a suitable solvent, such as acetone, which is preferred.

The ratio of enantiomers, e.g. in the hydrogenation product of above described steps B) and/or a), can be determined by quantitative ³¹p NMR using enantiopure phosphite (8), prepared from (S)-BINOL and PCI₃ according to the procedure: [G. Francio, C. G. Arena, F. Faraone, C. Graiff, M. Lanfranchi, A. Tirpicchio, Eur. J. Inorg. Chem. 1999, 8, 1219-1228], as depicted in scheme 7 and explained below.

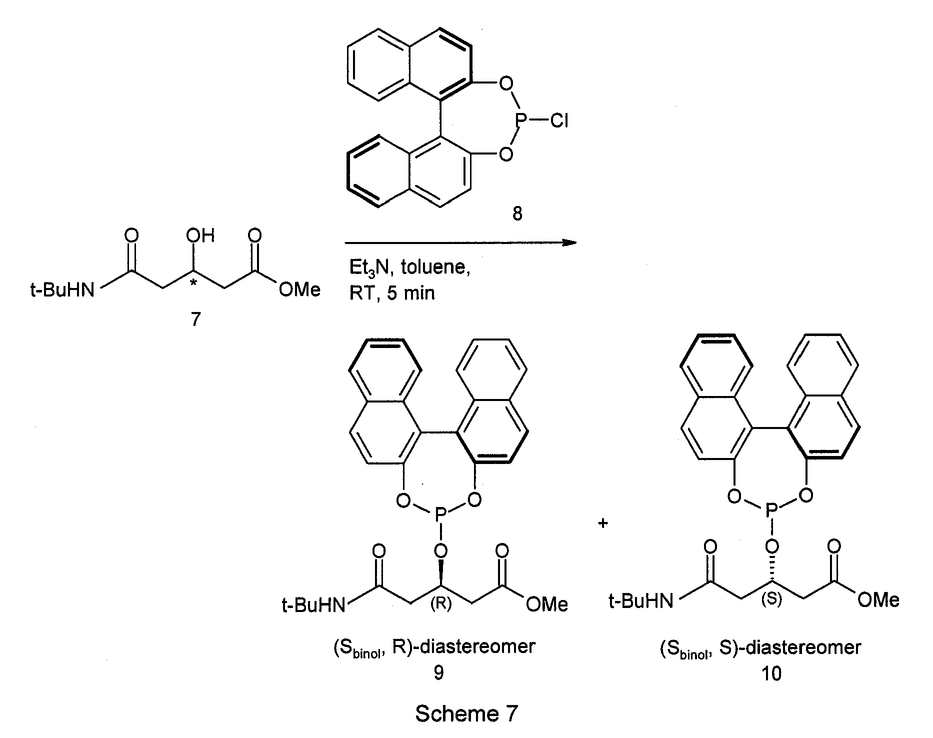

Scheme 7 shows the formation of two diastereomeric phosphites by reaction of alcohol 7 and chlorophosphite 8 prepared from (*S*)-BINOL and PCI₃.

The ³¹P NMR spectrum of both diastereomeric phosphite compounds (9 and 10) is characterized by signals separated till the base line (δ_{P} 152.5 and 154.9 ppm in C₆D₆ as solvent, δ_{P} 152.1 and 155.0 ppm in CDCl₃, or δ_{P} 151.4 and 154.7 ppm in toluene-d₈, respectively), therefore it is possible to precisely determine the enantiomeric composition of the catalytic hydrogenation product.

In a preferred embodiment of the process of the present invention R¹ and R² are different, more preferably the residues are significantly different in the space required. This enables an increase in the enantiomeric excesses obtained in the asymmetric hydrogenation reaction of step (a).

One advantage of the process of the present invention is that the compound of the formula I, i.e. the 3-hydroxy-pentanoic diacid derivatives can be easily obtained by reaction, in particular asymmetric hydrogenation, of the corresponding 3-oxo-pentanoic diacid derivatives or from reacting 5,5-dimethoxy-3-hydroxy-pentanoate derivatives in excellent enantiomeric excesses. Furthermore, the corresponding Wittig reagents, such as compound B of scheme 2 above, can be advantageously prepared from the 3-hydroxy-pentanoic diacid derivatives by the process of the present invention by reacting a compound of the formula 1.

Preferably, according to the present invention, the compound of the formula I is furthermore modified by a process comprising the step of reacting a compound of the formula I to obtain a compound of the formula VII 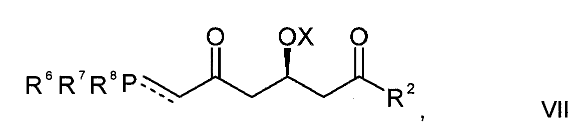 or a salt thereof, wherein R² and X are defined as above and R⁶, R⁷ and R⁸ are chosen such that the compound of formula VII is a Wittig reagent or a Horner-Wittig reagent.

How to choose the residues R⁶, R⁷ and R⁸ so that the compound of the formula VII is a Wittig reagent or a Horner-Wittig reagent or derivatives thereof is known to the person skilled in the art. Suitable selections of residues R⁶, R⁷ and R⁸ are exemplified in German patent application No. 10 2005 022 284.6, the disclosure of which is incorporated herein by reference. In particular in a usual Wittig reagent R⁶, R⁷ and R⁸ are phenyl residues and the bond of the phosphorus atom to the carbon chain is a double bond, and in a usual Horner-Wittig reagent R⁶ and R⁷ are both ethoxy residues and R⁸ is a oxygen, bound to the phosphorus atom by a double bond, i.e. R⁸ is a O= residue, and the phosphorus atom is bound to the carbon chain by a single bond. A Horner-Wittig reagent means a reagent to conduct a Horner-Wadsworth-Emmons-reaction, which is known in the art.

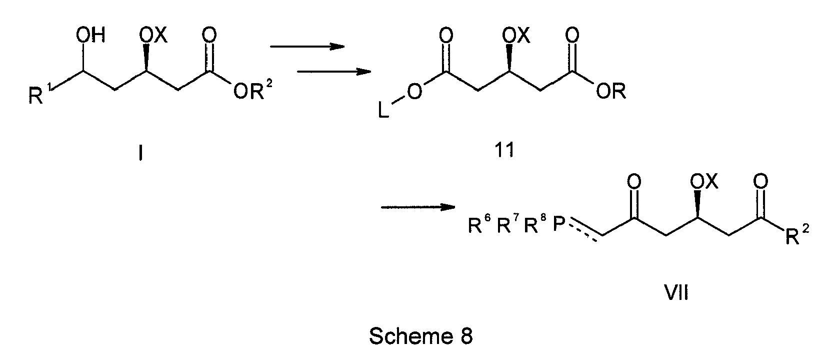

The reaction of the compound of formula I of the present invention to obtain the compound of the formula VII, i.e. the preparation of the Wittig reagent or the Horner-Wittig reagent from the 3-hydroxy-pentanoic diacid derivative, is carried out under usual conditions. Typically, a reaction sequence is applied (cf. Scheme 8), wherein in the first step a compound of the formula I is modified to obtain a compound with one free carboxylic acid, preferably such that residue R¹ is a OH group, followed by a usual activation of the carboxylic acid carbon atom to render it acceptable for a nucleophilic attack, e.g. a reaction with ETOC(O)CI in NEt₃, or a similar reaction, which methods are known to the person skilled in the art. Thereby e.g. a compound (11) is obtained, wherein L means a residue for activating the carboxyl group to which it is bound. After activation of the free carboxylic acid, the Wittig reagent or the Horner-Wittig reagent, respectively, can be prepared. as e.g. described in WO 03/087112 A1, WO 2006/091771 A2, or EP 554 455 A1, the disclosure of which documents is enclosed by reference herein. For Example, the Wittig reagent can be prepared by reaction of the activated carboxylic acid derivative of the 3-hydroxy-pentanoic diacid derivative (e.g. compound (11)) with methyltriphenylphosphoniumbromide to obtain a compound corresponding to compound B (cf. Scheme 1 above). Suitable solvents and reaction conditions are known in the art.

One advantage of the process of the present invention is that the compounds of the formula VII, which can advantageously be obtained by reacting a compound of formula I of the present invention, which preferably has a protected hydroxyl group at the 3-position, can also be prepared with excellent enantiomeric excess and application of industrially applicable reaction steps, compared to known processes. The compounds of the formula IV, which are Wittig reagents or Horner-Wittig reagents, respectively, can be advantageously used within a Wittig reaction or a Horner-Wittig reaction, respectively, to obtain a substituted pyrimidine derivative as described above, in particular rosuvastatin.

Preferably, according to the present invention in the process of the present invention, the compound of formula VII is further reacted with a compound of formula VIII 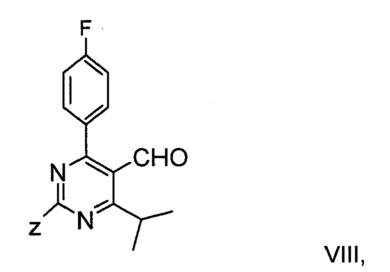 wherein Z is a -NMeSO₂Me group or a group capable of being converted into a - NMeSO₂Me group, to obtain a compound of the formula IX 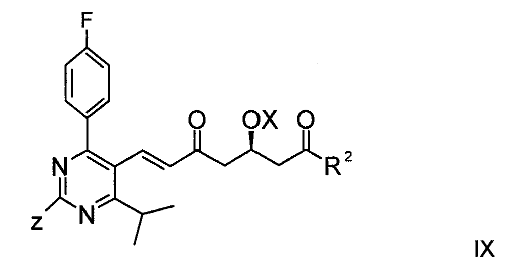 or a salt thereof, wherein R², X and Z are defined as above.

Residue Z is a -NMeSO₂Me group or a group capable of being converted into a - NMeSO₂Me group. The term -NMeSO₂Me group means a residue as depicted in the following formula XI

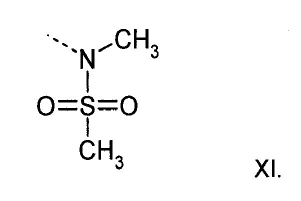

Groups capable of being converted into a -NMeSO₂Me group means that the group is selected from any functional group which can be converted, by carrying out one or more chemical steps, to form a -NMeSO₂Me group. Suitable groups which are capable of being converted, and the chemical synthesis steps that can be used to carry out the conversion are well known in the art, and are e.g. described in WO 2006/067456, the disclosure of which is incorporated herein by reference. Preferred groups capable of being converted into a NMeSO₂Me group are hydroxy, C₁₋₁₀ alkoxy, halogen (in particular chloro), tosyloxy, amino, C₁₋₁₀ alkylamino, such as methylamino, C₁₋₁₀ dialkylamino and methyl sulfonylamino groups.

The reaction of the compound of the formula VII with a compound of the formula VIII of the present invention, i.e. the Wittig reaction or the Horner-Wittig reaction can be conducted in solvents and under conditions as usually applied and known in the art. As suitable solvents each solvent used to conduct the Wittig reaction or the Horner-Wittig reaction, respectively, can be used, preferably an apolar and aprotic solvent, such as MeCN or toluene, which are preferred. The reaction is typically conducted until completion, e.g. for 4 to 48 hours.

The compound of the formula VIII is obtainable by known processes, as e.g. described in WO 03/097614, the disclosure of which is incorporated herein by reference.

The process of the present invention can be furthermore modified by hydrogenation and optionally deprotecting and/or protecting any protected or unprotected group of a compound of the formula IX, obtained by the above described process, in order to obtain a compound of the formula X 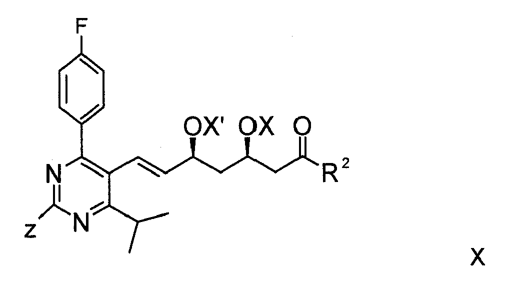 or a salt thereof, wherein X' is H or a hydroxy protecting group and X, R² and Z are defined as above.

How to conduct the hydrogenating reaction and the optionally deprotecting and/or protecting reactions to obtain a compound of the formula X by reacting a compound of the formula IX is known to the person skilled in the art. It is also known how to deprotect and/or protect any protected or unprotected group of the concerned compounds. Typically silicium containing protecting groups are removed by using an aqueous solution of HF, e.g. using MeCN as solvent. The hydrogenating reaction is typically conducted using a compound of the formula IX, wherein X is hydrogen by reacting such compound with a boron-containing reducing agent, e.g. Et₂BOMe and NaBH₄ in a suitable solvent. Further suitable reducing agents, in particular such to obtain the stereo chemistry of the compound of formula X as indicated, and the suitable reaction condition are known in the art.

Preferably, the compound of the formula X is modified such that X' and X are both hydrogen, R² is OH and Z is a -NMeSO₂Me group, such that the compound of the formula X is rosuvastatin.

In one preferred embodiment in the process of the present invention Z is -NMeSO₂Me or Z is converted into a -NMeSO₂Me group prior to reaction of the compound of the formula VIII with a compound of the formula Vil, and is most preferably such process that in the compounds of the formulas IX and X Z is also a -NMeSO₂Me group.

In one embodiment of the present invention the process can be modified such that the compound of the formula I has the formula I' 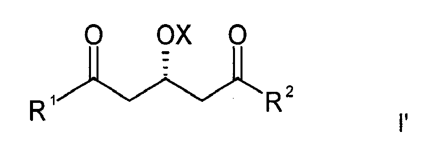 wherein residues R¹, R² and X are defined as above. It is known to the person skilled in the art how to adopt the hydrogenation conditions, in particular how the chiral catalyst has to be modified or to be chosen in particular in steps a) and/or B) such that the compound of the formula I' is obtained. Preferably also the compounds of the formulas III-VII are modified accordingly.

The present invention also relates to a compound of the formula I" 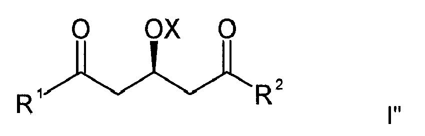 wherein X is H or a hydroxy protecting group, and R¹ and R² are independently selected from OH, OR³, wherein R³ is a carboxyl protecting group, or NR⁴R⁵, wherein R⁴ and R⁵ are independently H or an amido protecting group, with the proviso that at least one residue R¹ or R² is NR⁴R⁵, wherein R⁴ and R⁵ are independently H or an amido protecting group. These compounds are useful in the preparation of rosuvastatin.

The present invention further relates to the use of a compound of the formula I as described above for the preparation of rosuvastatin.

The present invention further relates to the use of a compound of the formula IV 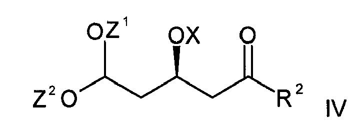 wherein Z¹ is a hydroxy protecting group, Z² is a hydroxy protecting group, X is H or a hydroxy protecting group and R² is selected from OH, OR³, wherein R³ is a carboxyl protecting group, or NR⁴R⁵, wherein R⁴ and R⁵ are independently H or an amido protecting group, for the preparation of rosuvastatin, and the use of a compound of the formula IV' 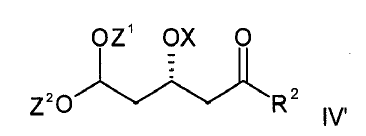 wherein Z¹ is a hydroxy protecting group, Z² is a hydroxy protecting group, X is H or a hydroxy protecting group and R² is selected from OH, OR³, wherein R³ is a carboxyl protecting group, or NR⁴R⁵, wherein R⁴ and R⁵ are independently H or an amido protecting group, for the preparation of rosuvastatin.

Within this application, all starting materials, intermediates and products to be used in the processes of the present invention may be used as racemates or enantiomerically enriched mixtures, e.g. mixtures which are enriched in one enantiomer or comprise only one substantially purified enantiomer.

Each process of the present invention can further comprise one or more steps of separation or enrichment of enantiomers, e.g. steps of racemic separation. Methods of separation or enrichment of enantiomers are known in the art.

Preferably, the stereo configuration of starting materials, intermediates and products is chosen such that when used in processes of the present invention the intermediates and products resulting from said processes show the stereo configuration suitable for the preparation of rosuvastatin or are in or correspond to the stereo configuration of rosuvastatin.

The present invention will now be further illustrated by the following examples which are not intended to be limiting.

MeOH and AcOEt (packed under N₂) for hydrogenation were purchased from Aldrich. CH₂Cl₂ was distilled over CaH₂, THF and Et₂O were distilled over Na-Ph₂CO under Ar, DMF was dried over 3A molecular sieves for 72 h prior to vacuum distillation. Other commercial reagents were used without additional purification. NMR spectra were recorded with a Bruker ARX 400 spectrometer or Bruker ARX 300 spectrometer. Chemical shifts (δ, in ppm) are given relative to TMS as internal standard for¹H NMR and relative to the residual CDCl₃ peak for ¹³ C NMR (δ = 77.16 ppm) and C₆D₆ peak for ¹³C NMR (δ = 128.06). Spin-spin coupling constants (*J*) are given in Hz. The optical rotation was measured on a "gyromat-HP" instrument.

### Example 1 - Preparation of methyl N-tert-butyl-3-oxoglutarate (6)

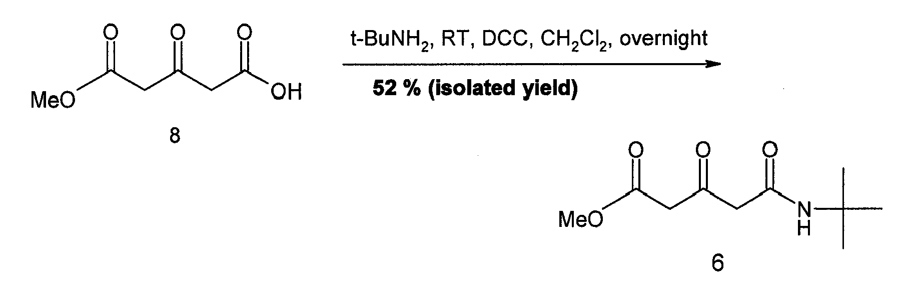

Methyl N-tert-butyl-3-oxoglutarate (6) was prepared by acylation of tert-BuNH₂ by monomethyl acetonedicarboxylate (8) in the presence dicyclohexyl carbodiimide (DCC) in dichloromethane at room temperature (RT). Methyl N-tert-butyl-3-oxoglutarate (6) was obtained in 52 % yield.

### Example 2 - Asymmetric hydrogenation of methyl 5-(tert-butylamino)-3,5-dioxopentanoate

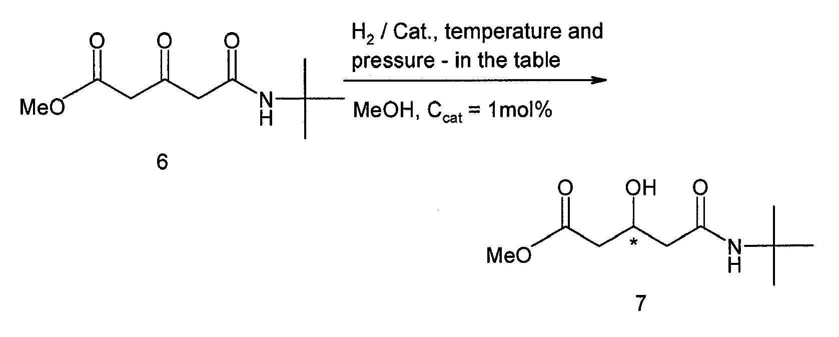

| Entry | H₂ Pressure → | Pressure: 5 bar | Pressure: 10 bar | Pressure: 25 bar | | Pressure: 50 bar |
|---|---|---|---|---|---|---|
| | Temperature °C: → | 25°C* | 25°C | 25°C | 50°C | 25°C |
| | Catalyst ↓ | Enantiomeric excess (ee), % [conversion, %] | | | | |
| 1. | (*S*)-(-)- [Ru(BINAP)(p-cymene)CI]CI | 96 % [>99] | 90 % [>99] | 90 % [>99] | 87 % [>99] | 84 % [>99] |
| 2. | (*R*)-(+)- [Ru(BINAP)(p-cymene)CI]CI | 94 % [>99] | 90 % [>99] | 88 % [>99] | 68 % [>99] | 83 % [>99] |
| 3. | (*R*)-(+)- [Ru(BINAP)]Cl₂ | - | 93 % [>99] | 88 % [>99] | 92 % [>99] | 82 % [>99] |
| 4. | (*R*)-(+)-[Ru(Tol- BINAP)]Cl₂ | - | 87 % [>99] | - | - | 28 % [>99] |
| 5. | 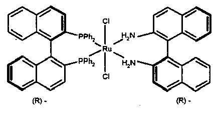 | 96 % [>99] | 95 % [>99] | 91 % [>99] | 72 % [>99] | 68 % [>99] |
| 6. | 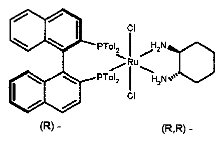 | - | 88 % [>99] | 88 % [>99] | - | 80 % [>99] |

| | | | | | | |
|---|---|---|---|---|---|---|
| * Conversions were detected by ¹H NMR after reaction time of 69h | | | | | | |

The above table shows the enantiomeric excesses and the conversion rates of the asymmetric hydrogenation of methyl 5-(*tert*-butylamino)-3,5-dioxopentanoate (6) conducted at indicated temperatures and different hydrogen pressures under application of different catalysts.

**The catalytic asymmetric hydrogenation of 6 was conducted following the general procedure**: Keto ester **6** (1.72 g, 8 mmol) and corresponding chiral complex as indicated in the table, amount about 0.08 mmol, were placed in an autoclave under argon followed by addition of abs. MeOH (8 mL). The mixture was pressurized with hydrogen to pressures indicated in the table (5, 10, 25 or 50 bar) and stirred at the indicated temperature until the H₂ consumption stopped (4 h). Evaporation of the solvent in vacuum gave **7** as yellowish viscous oil in quantitative yield.
¹H-NMR (400 MHz, CDCl₃): δ = 1.30 (s, 9H, C(C*H*₃)₃), 2.16-2.42 (m, 4H, C*H*₂CO₂Me and C*H*₂C(O)NH-*tert*-Bu), 3.25 (s, 3H, CO₂C*H*₃), 3.48 (br. s, 1H, O*H*), 4.38-450 (m, 1H, C*H*OH).

**The determination of the enantiomeric composition of compound 7 was conducted following the general procedure (according to Scheme 7):** A 10 % solution of (*S*)-**8** (0.5 mL, 0.14 mmol) in dry benzene, the alcohol **7** (21 mg, 0.10 mmol), abs. Et₃N (20 mg, 0.19 mmol) and dry d₆-benzene (0.1 mL) were mixed together in a vial. The resultant pale solution was immediately transferred into an NMR-tube under argon and then the ³¹P NMR spectrum was recorded. Two singlet peaks at δ_{P} 152.5 and 154.9 ppm separated till the baseline correspond to compounds **9** and **10**, respectively. The enantiomeric excess of the alcohol **7** was calculated from the integral intensities of the peaks.

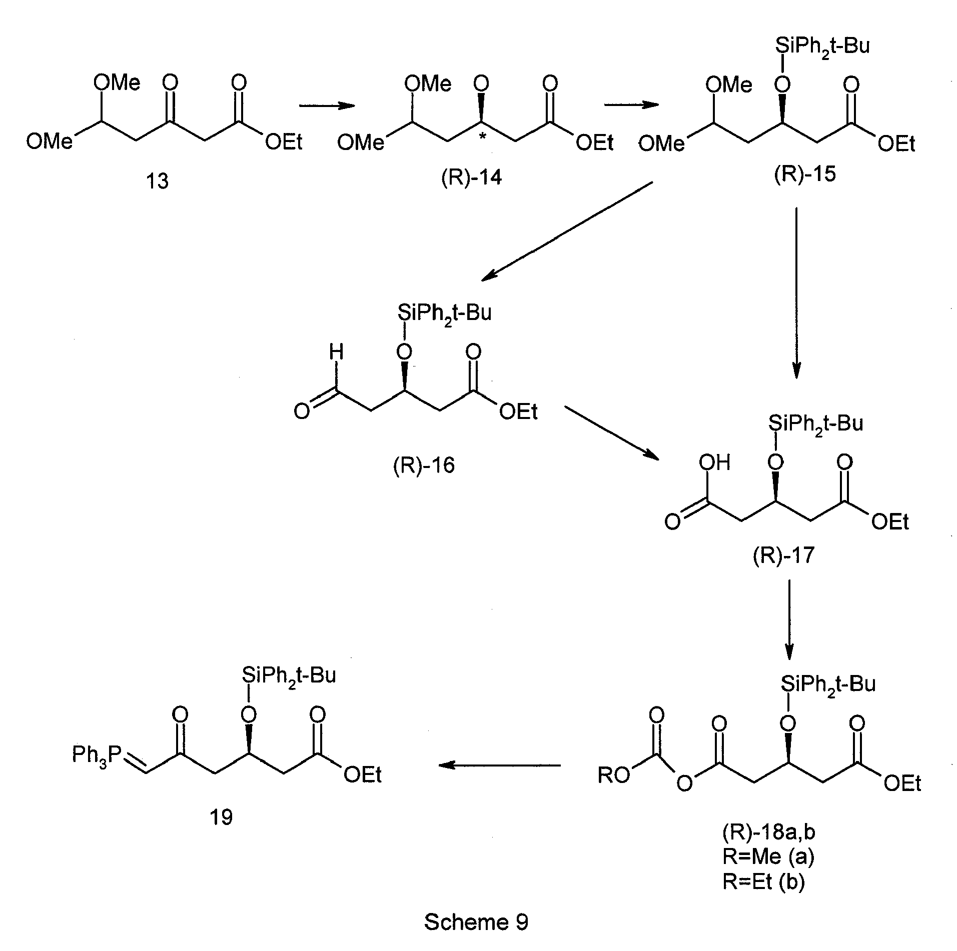

### Example 3: Ethyl 5,5-dimethoxy-3-oxo-pentanoate (13)

Solid N,N'-carbonyldiimidazole (Im₂CO) (50.02 g, 0.308 mol) was added in portions during 5-10 min to a solution of acid **12** (41.45 g, 0.288 mol) in THF (200 ml) (gas evolution). Then the mixture was stirred at RT for 2 h to produce *in situ* the corresponding imidazolide. In another flask, a mixture of monoethyl malonate (83.5 g, 0.632 mol) and commercial Mg powder with particle size <0.1 mm (10.24 g, 0.422 mol) in THF (300 ml) was stirred under reflux for 4 h to produce *in situ* Mg(OOCCH₂COOEt)₂, which was cooled down to RT and added to the former solution. The residual Mg powder was rinsed with THF (100 ml). The wash solution was added to the reaction mixture. The mixture was stirred overnight at RT and concentrated in vacuum. The residue was dissolved in ethyl acetate (300 ml) and acidified with 2M NaHSO₄ (620 ml) under vigorous stirring. The organic layer was separated, washed successively with water, sat. NaHCO₃ (3x200 ml) and brine. It was subsequently dried over Na₂SO₄ and evaporated. Vacuum distillation of the residue (b.p. 86-92 °C/0.06 mbar) afforded 53.62 g (91 % yield) of keto ester **13**. ¹H-NMR (CDCl₃): 1.28 (t, *J* 7.1Hz, 3H), 2.86 (d, *J* 5.6Hz, 2H), 3.37 (s, 6H), 3.49 (s, 2H), 4.20 (q, *J* 7.1Hz, 2H), 4.78 (t, *J* 5.6Hz, 1H).

### Example 4: Ethyl 5,5-dimethoxy-3-hydroxy-pentanoate (14)

### Catalytic hydrogenation of 13

Keto ester **13** (2.04 g, 10 mmol) and Ru((*R*)-BINAP)Cl₂ (0.004 g, 0.005 mmol) were placed in an autoclave under argon followed by addition of abs. MeOH (8 ml). The mixture was pressurized with hydrogen to 50 bar and stirred at 50 °C until the H₂ consumption stopped (1 h). Evaporation of the solvent in vacuum gave hydroxy ester **14** as yellowish oil in quantitative yield. ¹H-NMR (CDCl₃): 1.27 (t, *J* 7.1 Hz, 3H), 1.75-1.83 (m, 2H), 2.46-2.51 (m, 2H), 3.22 (br., 1H), 3.37 (s, 6H), 4.17 (q, *J* 7.1 Hz, 2H), 4.15-4.23 (m, 1H), 4.61 (t, *J* 5.7Hz, 1H). ¹³C-NMR (CDCl₃): 14.6, 39.4, 42.0, 53.6, 53.9, 61.0, 65.3, 103.3, 172.6.

### Determination of the enantiomeric composition of compound 14 (in accordance to scheme 7)

A 10% solution of (*S*)-8 ((0.5 ml, 0.14 mmol) in dry toluene, the alcohol **14** (21 mg, 0.10 mmol), abs. Et₃N ((20 mg, 0.19 mmol) and dry d₆-benzene (0.1 ml) were mixed together in a vial. The resultant pale solution was immediately transferred into an NMR-tube under argon and then the ³¹P NMR spectrum was recorded. Two singlet peaks at δ_{P} 151.4 and 154.6 separated till the baseline correspond to (S_{Binol},S)-diastereomer and (S_{Binol},R)-diastereomer, respectively. The enantiomeric excess of the alcohol **14** was calculated from the integral intensities of the peaks.

### Example 5: Ethyl (R)-3-(tert-butyldiphenylsilyloxy)-5,5-dimethoxypentanoate (15, compound of the formula IV):

To a solution of ethyl (*R*)-3-hydroxy-5,5-dimethoxypentanoate ((*R*)-**14**) (2.83 g, 13.72 mmol) and imidazole (2.05 g, 30.18 mmol) in 15 mL of dry DMF *tert-*butyldiphenylsilylchloride (4.15 g, 15.09 mmol) was added dropwise during 30 min at 0 °C. The reaction mixture was stirred for 2 h at RT and then diluted with 100 mL of H₂O. The crude product was extracted with ethyl acetate (3×100 mL) and the combined extracts were washed successively with water, saturated NaHCO₃ (2×100 mL), brine and dried over Na₂SO₄. Then EtOAc was evaporated. Column chromatography purification (silica gel, eluent *n*-hexane/EtOAc = 5:1) afforded 5.49 g (90% yield) of (*R*)-**15**. ¹H-NMR (400 MHz, C₆D₆): δ = 0.9 (t, *J* = 7.17 Hz, 3H, OCH₂C*H*₃), 1.17 (s, 9H, C(C*H*₃)₃), 2.01-2.06 (m, 2H, C*H*₂CH(OMe)₂), 2.57 (d, *J* = 1.16 Hz, 1H, C*H*ₐH_{b}CO₂Et), 2.59 (d, *J* = 1.26 Hz, 1H, CHₐ*H*_{b}CO₂Et), 2.95 (s, 3H, CH(OC*H*₃)ₐ(OCH₃)_{b}), 2.99 (s, 3H, CH(OCH₃)ₐ(OC*H*₃)_{b}), 3.87 (q, *J* = 7.17 Hz, 2H, OC*H*₂CH₃), 4.54 (t, *J* = 5.71 Hz, 1H, C*H*(OCH₃)₂), 4.60 (quintet, *J* = 6.11 Hz, 1H, C*H*OSi), 7.17-7.21 (m, 6H, Ar), 7.78-7.86 (m, 4H, Ar). ¹³C-NMR (75 MHz, CDCl₃,): δ = 14.19 (OCH₂CH₃), 19.21 (CMe₃), 26.98 (C(CH₃)₃), 37.87 (*C*H₂CO₂Et), 42.40 (*C*H₂CH(OMe)₂), 52.05 (CH(OCH₃)*ₐ*(OCH₃)_{b}), 52.90 (CH(OCH₃)ₐ(OCH₃)_{b}), 60.36 (O*C*H₂Me), 67.50 (*C*HOSi), 101.58 (CH(OMe)₂), 133.76 (*C*, Ar), 133.95 (*C*, Ar), 134.92 (*C*H, Ar), 135.49 (*C*, Ar), 135.60 (*C*H, Ar), 135.97 (*C*H, Ar), 136.04 (*C*H, Ar), 171.26 (CO₂Et).

### Example 6: Ethyl (R)-3-(tert-butyldiphenylsilyloxy)-5-oxopentanoate (16, compound of the formula VI):

To a solution of (*R*)-**15** (1.2 g, 2.7 mmol) in acetone/H₂O (5:1, 28.8 mL) *p*-toluenesulfonic acid hydrate (*p*-TsOH×H₂O) (120 mg, 0.63 mmol) was added. The reaction mixture was refluxed for 2 h until 100% of conversion was achieved (reaction was controlled by TLC: toluene/EtOAc = 20:1) and the solvent was evaporated under reduced pressure. The residue was dissolved in EtOAc, washed successively with saturated NaHCO₃ and brine. The organic layer was subsequently dried over Na₂SO₄ and evaporated. Purification of the crude product by column chromatography (silica gel, eluent *n*-hexane/EtOAc = 10:1) afforded 1.05 g (98% yield) of aldehyde (*R*)-**16** as a colorless viscous oil. ¹H-NMR (300 MHz, C₆D₆): δ = 0.88 (t, *J* = 7.17 Hz, 3H, OCH₂C*H*₃), 1.11 (s, 9H, C(C*H*₃)₃), 2.30-2.34 (m, 2H, C*H*₂CHO), 2.39 (d, *J* = 6.07 Hz, 1H, C*Hₐ*H_{b}CO₂Et), 2.46 (d, *J* = 6.07 Hz, 1H, CHₐ*H_{b}*CO₂Et), 3.82 (q, *J* = 7.17 Hz, 2H, OC*H*₂CH₃), 4.67 (quintet, *J* = 6.0 Hz, 1H, C*H*OSi), 7.17-7.23 (m, 6H, Ar), 7.67-7.80 (m, 4H, Ar), 9.24 (t, *J* = 1.88 Hz, 1H, C*H*O).

### Example 7: (R)-3-(tert-butyldiphenylsilyloxy)-5-ethoxy-5-oxopentanoic acid (17, compound of the formula V):

To a solution of alcohol (*R*)-**15** (1.2g, 2.7 mmol) in acetone/water mixture (5:1, 10.6 mL) Jones' oxidizing reagent was added dropwise (during ca. 30 min) at 0 °C. The addition was continued until the characteristic orange color of the reagent persisted for about 20 min. The stirrer was removed and the mixture decanted. The residual green salts were rinsed with acetone (2×10 mL). The washings were added to the main acetone solution and additional oxidizing agent was added, if necessary, to ensure complete reaction. The stirrer was replaced and 2-propanol was added dropwise until excess of Jones' reagent was destroyed. In small portions and with caution a solution of NaHCO₃ was added and the suspension was stirred vigorously until the pH of the reaction mixture became neutral. The suspension was filtered and the filter cake was washed with acetone (2x25 mL). The filtrate was concentrated and additionally purified by column chromatography (silica gel, eluent n-hexane/EtOAc = 1:1). Yield of (*R*)-**17** 761 mg (68% yield) as colorless oil. ¹H-NMR (300 MHz, CDCl₃): δ = 0.92 (s, 9H, SiC(C*H*₃)₃), 1.08 (t, *J* = 7.17 Hz, 3H, OCH₂C*H*₃), 2.38-2.60 (m, 4H, C*H*₂CO₂H and C*H*₂CO₂Et), 3.92 (q, *J* = 7.17 Hz, 2H, OC*H*₂CH₃), 4.67 (quintet, *J* = 6.04 Hz, 1H, C*H*OSi), 7.23-7.36 (m, 6H, Ar), 7.54-7.60 (m, 4H, Ar), 12.9 (br. 1H, CO₂*H*). ¹³C-NMR (75 MHz, CDCl₃): δ = 14.12 (OCH₂CH₃), 19.25 (*C*Me₃), 26.83 (C(*C*H₃)₃), 41.43 (*C*H₂CO₂Et), 41.68 (*C*H₂CO₂H), 60.55 (O*C*H₂Me), 66.98 (*C*HOSi), 127.69 (*C*H, Ar), 129.89 (*C*H, Ar), 133.24 (*C*, Ar), 133.36 (*C*, Ar), 135.89 (*C*H, Ar), 170.78 (CO₂Et), 176.78 (CO₂H).

### Example 8: (R)-3-(tert-butyldiphenylsilyloxy)-5-ethoxy-5-oxopentanoic (ethyl carbonic) anhydride (18b):

A solution of acid (*R*)-**17** (4.97 g, 11.99 mmol) and triethylamine (1.81 g, 17.89 mmol) in dry toluene (40 mL) was cooled to -40 °C, and ethyl chlorocarbonate (1.95 g, 17.97 mmol) was added dropwise. The reaction mixture was allowed to warm to 0 °C and stirred for 1 h. Then the reaction mixture was stirred overnight at RT and diluted with ethyl acetate (150 mL). The solution was washed successively with saturated NaHCO₃ (2×80 mL), brine (2×80 mL) and then dried over MgSO₄. The solvent was evaporated and the residue was dried in high vacuum to give 5.54 g (95% yield) of the anhydride (*R*)-**18b** as a clear colorless oil. ¹H-NMR (400 MHz, C₆D₆): δ = 0.87 (t, *J* = 7.17 Hz, 3H, OCH₂C*H*₃), 0.90 (t, *J* = 7.17 Hz, 3H, OCH₂C*H*₃), 1.14 (s, 9H, SiC(C*H*₃)₃), 2.49-2.72 (m, 4H C*H*₂CO₂CO₂Et and C*H*₂CO₂Et), 3.82 (q, *J* = 7.17 Hz, 2H, OC*H*₂CH₃), 3.89 (q, *J* = 7.17 Hz, 2H, OC*H*₂CH₃), 4.69 (quintet, *J* = 6.02 Hz, 1H, CHOSi), 7.17-7.26 (m, 6H, Ar), 7.74-7.85 (m, 4H, Ar).

### Example 9: Ethyl (R)-3-(tert-butyldiphenylsilyloxy)-5-oxo-6-(triphenylphosphoranylidene)hexanoate (19, compound of the formula VII):

A suspension of methyltriphenylphosphonium bromide (8.85 g, 24.77 mmol) in dry THF (40 mL) was cooled to -78°C, and 1.6 M *n*-BuLi (15.4 mL, 24.66 mmol) in hexane was added dropwise over 20 min. The mixture was allowed to warm to 0 °C and kept at this temperature for ca. 10 min. (solution became yellow and the white powder was dissolved). The mixture was cooled again to -78°C, and a THF solution of the anhydride **18b** (6.0 g, 12.33 mmol) was added over 1 h. The resulting mixture was stirred overnight at -30°C, warmed up to RT and stirred additionally for 1 h. The mixture was poured into water and extracted with EtOAc (2×100 mL). Combined organic layers were washed with saturated NaHCO₃ brine, and dried over MgSO₄. Solvent was evaporated and residual material was purified by column chromatography (silica gel, *n*-hexane/EtOAc = 1:10) to obtain 3.7 g (45%) of Wittig reagent **19** as viscous oil. [α]_{D}²² = -6.5 (c 1, CHCl₃)₋ ¹H-NMR (400 MHz, CDCl₃): δ = 1.01 (s, 9H, SiC(C*H*₃)₃), 1.19 (t, *J* = 7.12 Hz, 3H, OCH₂C*H*₃), 2.33-2.83 (m, 4H, C*H*₂COCH=PPh₃ and C*H*₂CO₂Et), 3.43 (br., 1H, C*H*=P), 3.96-4.13 (m, 2H, OC*H*₂CH₃), 4.57-4.65 (m, 1H, C*H*OSi), 7.25-7.33 (m, 6H, Ar), 7.37-7.44 (m, 6H, Ar), 7.47-7.55 (m, 9H, Ar) 7.69-7.74 (m, 4H, Ar). ¹³C-NMR (100 MHz, CDCl₃,): δ = 14.11 (OCH₂CH₃), 19.23 (CMe₃), 26.83 (C(CH₃)₃), 42.24 (CH₂CO₂Et), 49.44 (d, *J*_{CP} = 14.7 Hz, CH₂COCH=PPh₃), 53.3 (d, *J*_{CP} = 106.5 Hz, HC=PPh₃), 59.99 (OCH₂Me), 70.55 (CHOSi), 126.27 (*C*, Ar), 127.17 (*C*H, Ar), 127.34 (*C*, Ar), 127.44 (*C*, Ar), 127.46 (*C*, Ar), 128.51 (*C*, Ar), 128.62 (*C*H, Ar), 128.74 (*C*H, Ar), 129.34 (d, *J*_{CP} = 4.55 Hz, *C*H, Ar), 131.95 (d, *J*_{CP} = 2.89 Hz, *C*H, Ar), 132.93 (*C*H, Ar), 133.03 (*C*H, Ar), 133.91 (*C*, Ar), 134.36 (*C*, Ar), 135.92 (d, *J*_{CP} = 1.59 Hz, *C*H, Ar), 172.21 (CO₂Et), 189.58 (COCH=PPh₃). ³¹P-NMR (162 MHz, CDCl₃,): δ = 15.25.

## Claims

1. Process for the preparation of a compound of the formula I 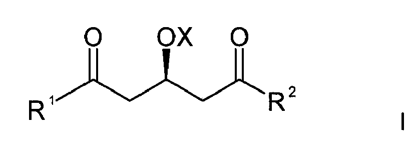 or a salt thereof, wherein X is H or a hydroxy protecting group, and R¹ and R² are independently selected from OH, OR³, wherein R³ is a carboxyl protecting group, or NR⁴R⁵, wherein R⁴ and R⁵ are independently H or an amido protecting group,
which process comprises the steps of
b) reacting a compound of the formula IV 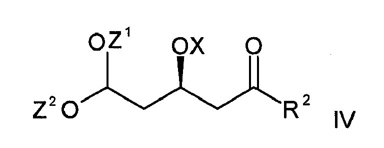 or a salt thereof,
wherein Z¹ is a hydroxy protecting group, Z² is a hydroxy protecting group, and X and R² are defined as above, to obtain a compound of formula V 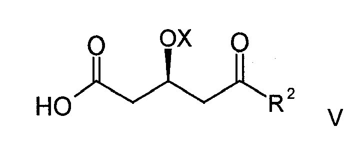 or a salt thereof,
wherein X and R² are defined as above, and
c) optionally converting the residues of the compound of the formula V obtained from step b) to obtain the compound of the formula I.

2. Process according to claim 1, wherein reaction step b) comprises the step of oxidating the compound of formula IV 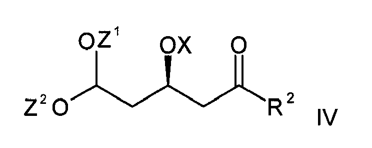 or a salt thereof,
wherein Z¹ is a hydroxy protecting group, Z² is a hydroxy protecting group, and X and R² are defined as in claim 1,
to obtain a compound of formula V 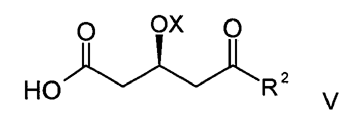 or a salt thereof,
wherein X and R² are defined as above.

3. Process according to claim 1, wherein the reaction step b) comprises the step of hydrolysing the compound of the formula IV 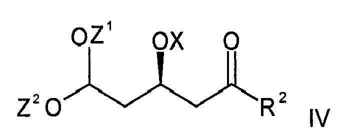 or a salt thereof,
wherein Z¹ is a hydroxy protecting group, Z² is a hydroxy protecting group, and X and R² are defined as above, to obtain a compound of formula VI 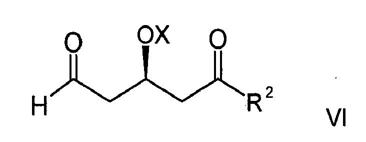 or a salt thereof,
wherein X and R² are defined as above, and the step of oxidating the compound of formula VI to obtain a compound of formula V 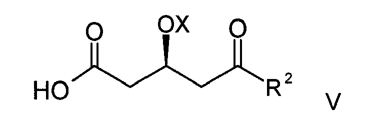 or a salt thereof,
wherein X and R² are defined as above.

4. Process according to any of claims 1-3, wherein R¹ is OR³ and R³ is alkyl, aryl or aralkyl, preferably R³ is a C₁₋₆-alkyl group, R² is NR⁴R⁵ and R⁴ is alkyl, aryl or an aralkyl group, preferably R⁴ is a C₁₋₆-alkyl group, R⁵ is H or an alkyl, aryl or an aralkyl group, preferably R⁵ is H, and X is H or a hydroxy protecting group.

5. Process according to any of claims 1-3, wherein R¹ and R² are OR³ and R³ is alkyl, aryl or aralkyl, preferably R³ is a C₁₋₆ alkyl group, X is H or a hydroxy protecting group and Z¹ and Z² are a hydroxy protecting group.

6. Process according to any of the claims 1-5, further comprising the step of reacting a compound of formula I to obtain a compound of the formula VII 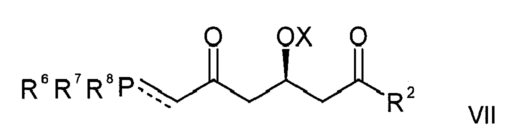 or a salt thereof, wherein R²and X are defined as in claim 1 and R⁶, R⁷ and R⁸ are chosen such that the compound of formula VII is a Wittig reagent or a Horner-Wittig reagent.

7. Process according to claim 6, further comprising the step of reacting the compound of the formula VII with a compound of the formula VIII 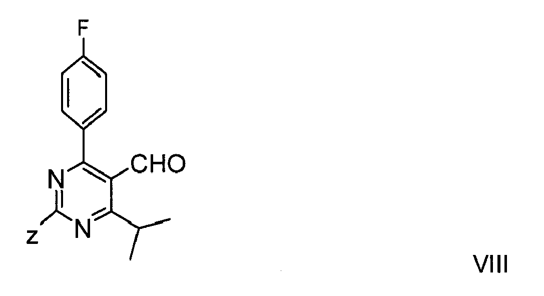 wherein Z is a -NMeSO₂Me group or a group capable of being converted into a - NMeSO₂Me group,
to obtain a compound of the formula IX 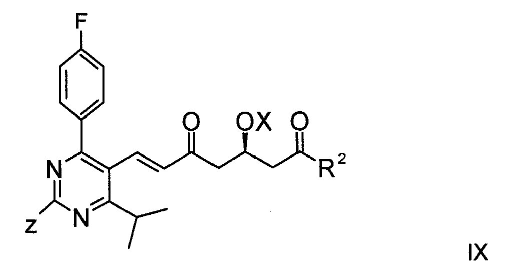 or a salt thereof, wherein R² and X are defined as in claim 6 and Z is defined as above.

8. Process according to claim 7, further comprising the step of hydrogenating and optionally deprotecting and/or protecting any protected or unprotected group of a compound of formula IX to obtain a compound of the formula X 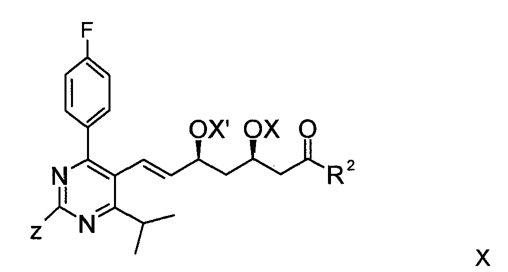 or a salt thereof, wherein X' is H or a hydroxy protecting group and X, R² and Z are defined as in claim 7.

9. Process according to claims 1-8, wherein the compound of the formula I has the formula I' 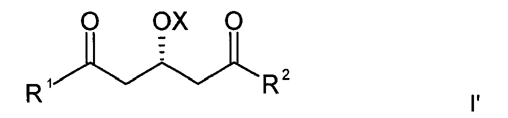 wherein R¹, R² and X are defined as in claim 1.

10. Use of a compound of the formula IV 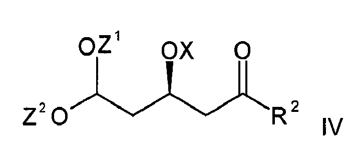 wherein Z¹ is a hydroxy protecting group, Z² is a hydroxy protecting group, X is H or a hydroxy protecting group and R² is selected from OH, OR³, wherein R³ is a carboxyl protecting group, or NR⁴R⁵, wherein R⁴ and R⁵ are independently H or an amido protecting group, for the preparation of rosuvastatin.

11. Use of a compound of formula IV' 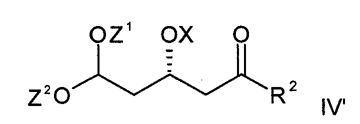 wherein Z¹ is a hydroxy protecting group, Z² is a hydroxy protecting group, X is H or a hydroxy protecting group and R² is selected from OH, OR³, wherein R³ is a carboxyl protecting group, or NR⁴R⁵, wherein R⁴ and R⁵ are independently H or an amido protecting group, for the preparation of rosuvastatin.
